# EUROPEAN PATENT APPLICATION

(11) **EP 1 538 219 A1**
(43) Date of publication of application: **08.06.2005**
(21) Application number: 03078855.8
(22) Date of filing: 04.12.2003
(51) Int. Cl.: C12Q 1/68

(54) **Method to diagnose or screen for type 1 diabetes mellitus**

(71) Applicant: Erasmus University Medical Center Rotterdam, 3015 GD Rotterdam (NL)
(72) Inventor: Drexhage, Hemmo Arjan, 3051 JD Rotterdam (NL); Ruwhof, Cindy, 2401 PJ Alphen aan den Rijn (NL)
(74) Representative: Prins, Adrianus Willem, Mr. Ir.

(57) **Abstract**

The invention relates to the field of medical diagnostics. More specifically, the invention relates to methods to diagnose or screen for Diabetes Mellitus type 1 (DM1) in a subject, preferably a human subject, by assaying for a marker for DM1. Provided is a method to diagnose or screen for DM1) in a subject, preferably a human subject, said method comprising determining the level of at least one, preferably at least two, more preferably at least three, most preferred at least four, DM1-specific gene product(s) in a biological sample isolated from said subject, preferably peripheral blood monocytes, wherein said DM1-specific gene is selected from the group comprising CCL2, MCP-3, HSPA1A, HSPA1B, EMP1, STX1A, BAZ1A, CD9, PTPN7, CDC42, FABP5, NAB2 and SDR1.

## Description

The invention relates to the field of medical diagnostics. More specifically, the invention relates to methods to diagnose or screen for Diabetes Mellitus type 1 (DM1) in a subject, preferably a human subject, by assaying for a marker for DM1.

Diabetes mellitus (DM), commonly referred to as "diabetes," is a chronic medical condition associated with abnormally high levels of glucose in the blood. Elevated levels of blood glucose (hyperglycemia) lead to spillage of glucose into the urine. Normally, blood glucose levels are tightly controlled by insulin, a hormone produced by the pancreas. Insulin lowers the blood glucose level. When the blood glucose elevates (for example, after eating food), insulin is released from the pancreas to normalize the glucose level. In patients with diabetes, the absence or insufficient production of insulin causes hyperglycemia. Insulin deficiency also causes an increased lipolysis (breakdown of body fat). During lipolysis, waste products called ketones are produced. Ketones are eliminated in the urine and through the lungs. Under normal conditions, the body can tolerate and eliminate ketones. But in diabetic ketoacidosis, fats are being broken down at such a high rate that the body can not eliminate the ketones fast enough and they build up in the blood. In high amounts, ketones are toxic to the body. They cause the acid-base balance to change and serious electrolyte and fluid imbalances result. Possible complications of ketoacidosis include depletion of potassium and phosphate, hypoglycemia, fluid accumulation in the brain and lungs, renal failure, heart failure, and death. Diabetes mellitus is a chronic medical condition, meaning it can last a lifetime. Over time, diabetes mellitus can lead to blindness, kidney failure, and nerve damage. Diabetes mellitus is also an important factor in accelerating the hardening and narrowing of the arteries (atherosclerosis), leading to strokes, coronary heart diseases, and other blood vessel diseases in the body. Narrowing of the arteries in the foot can lead to chronic ulcers and ultimately to amputation of the foot. Diabetes mellitus affects 12 million people (6% of the population) in the United States. The direct and indirect cost of diabetes mellitus is $40 billion per year. Diabetes is the third leading cause of death in the United States after heart disease and cancer.

There are two major types of diabetes mellitus, called type 1 or DM1 and type 2 or DM2. Approximately 10% of the patients with diabetes mellitus have DM1; the remaining 90% have DM2.

DM1 is also called insulin dependent diabetes mellitus (IDDM). In DM1, the insulin-producing pancreatic β-cells undergo an autoimmune attack by the body itself, and the pancreas is rendered incapable of making insulin. Europe has one of the highest prevalences with more than one million patients. The disease has devastating vascular complications with staggering costs; present treatment modalities are far from optimal, neither cure nor prevention being available. Individuals diagnosed with DM1 require regular insulin injections to survive. Islet cell transplants and pancreas transplants can in some cases eliminate the need for insulin injections. However, these procedures still require the patient to stay on a life-long schedule of antirejection medications.

DM2 usually develops in people older than 40 or people who are overweight (obese). In general, the treatment of DM2 patients does not involve insulin therapy but modification of certain lifestyle aspects (e.g. exercise, weight loss, a strict diet) and sometimes oral antidiabetics is sufficient to control blood glucose levels.

DM1 is frequently diagnosed in childhood, and is sometimes referred to as juvenile diabetes for that reason. Early diagnosis is important to prevent some of the more serious complications of diabetes, which include heart disease, blindness, high blood pressure, nerve damage, and kidney failure. However, although DM1 tends to occur most frequently in young, lean individuals, usually before 30 years of age, older patients do also present this form of diabetes. This type of DM1 is usually referred to as latent autoimmune diabetes of adulthood (LADA). Like the more common juvenile DM1, LADA is caused by immune-mediated destruction of the insulin-producing pancreatic beta cells. LADA is also known as slow-onset type 1 diabetes, late-onset autoimmune diabetes of adulthood, and type 1.5 diabetes. The main difference between juvenile DM1 and LADA is the age of diagnosis ― generally thirty years or older. Thus, DM1 can be present at any age, and the factors that determine the age of clinical manifestation are not known. DM2 (Adult-Onset Diabetes Mellitus) generally occurs when the body develops insulin resistance a result of genetic factors and obesity, and is typically diagnosed in adulthood. Insulin resistance causes hyperglycemia and, because of prolonged demand for insulin production, deterioration of the pancreatic beta cells. The combination of insulin resistance and decreased beta cell function ultimately causes DM2.

LADA is often misdiagnosed as DM2 because of the late age of onset. Like other forms of DM1, patients with LADA require insulin injections to normalize their blood glucose levels. To assume that obese patients over 30 years are inevitably DM2 cases is inappropriate, and can lead to incorrect treatment.

Therefore, when diagnosing diabetes, it is very important to be able to reliably discriminate between DM1 and DM2. Known diagnostic methods include a blood test for the presence (in DM1 patients) of islet cell antibodies (ICA), insulin auto-antibodies (IAA), and/or glutamic acid decarboxylase (a beta cell protein known as GAD) which can confirm a LADA diagnosis. However, the discriminative power (DM1 versus DM2) of test on the basis of the presence of antibodies is limited because DM1 patients are in up to 15% of the cases antibody-negative, whereas DM2 patients may, in up to 5% of the cases, be antibody-positive. The amount of c-peptide present, a protein that is generated during insulin production, can also be used to differentiate DM1 or LADA from DM2

Furthermore, these methods are only suitable for diagnosing cases of diabetes which are already apparent in a patient and do not allow for screening subjects who are at risk of developing DM1 (so-called "pre-diabetics"), for example first grade relatives of DM1-patients. Identification of subjects at risk for developing DM1 would for instance allow to initiate insulin- or other treatment before the clinical symptoms become apparent. Also, patients can be treated in an early stage to prevent (irreversible) side effects to occur e.g. the development of heart and/or vascular disease.

In view of the above, there is a clear need for improved and alternative methods for diagnosing and screening for DM1.

The invention now provides the insight that a number of genes are differentially expressed in patients suffering from DM1 when compared to healthy subjects or to DM2-patients. The differential expression level of 13 DM1-specific genes provides a basis for new clinically applicable tools to discriminate between DM1 and DM2 patients, in particular between LADA patients and DM2 patients, as well as to screen for patients who are at increased risk of developing DM1 i.e. predict DM1 development.

Provided herein is a method to diagnose or screen for Diabetes Mellitus type 1 (DM1) in a subject, preferably a human subject, said method comprising determining the level of at least one, preferably at least two, more preferably at least three, most preferred at least four, DM1-specific gene product(s) in a biological sample, preferably peripheral blood monocytes, isolated from said subject, wherein said DM1-specific gene product is selected from the group comprising CCL2, MCP-3, HSPA1A, HSPA1B, EMP1, STX1A, BAZ1A, CD9, PTPN7, CDC42, FABP5, NAB2 and SDR1. According to the invention, an elevated level of CCL2, MCP-3, EMP1, STX1A, CD9, PTPN7, CDC42, FABP5, NAB2 and/or SDR1 gene product(s) and/or a reduced level of HSPA1A, HSPA1B and/or BAZ1A gene product(s) compared to the level of said gene product(s) in a sample of a healthy subject or of DM2-patient is indicative of DM1 or an increased risk of developing DM1. More preferably, said DM1-specific gene product is selected from the group comprising STX1A, BAZ1A, CD9, PTPN7, FABP5 and SDR1. Thus, a method is provided for the diagnosis or detection of DM1 in a subject by assaying for a marker of DM1. Such a method is of very useful for diagnosing and/or monitoring the development of preclinical stages of DM1 in a patient and thus presents a valuable opportunity to initiate preventive or ameliorative treatment of DM1.

CCL2 refers to chemokine (C-C motif) ligand 2, also known as monocyte chemotactic protein 1 (MCP1), small inducible cytokine A2 (SCYA2) or monocyte chemotactic and activating factor (MCAF). The GenBank accession numbers of CCL2 are M28225 en M26683. MCP-3 refers to monocyte chemotactic protein 3, also known as chemokine (C-C motif) ligand 7 (CCL7) or small inducible cytokine A7 (SCA7). The accession number of MCP-3 is X72308. HSPA1A and HSPA1B refer to the 70 kD heat shock protein (Hsp70) 1A and 1B, respectively. They are also known as MHC class III HSP70-1 and HSP70-2. The accession number are M11717 (HSPA1A) and M59830 (HSPA1B). EMP1refers to epithelial membrane protein 1, also known as tumor associated membrane protein (TMP). The accession number is U43916. STX1A refers to Syntaxin 1A with accession number L37792. BAZ1A refers to bromodomain adjacent to zinc finger domain, also known as williams syndrome transcription factor-related chromatin remodelling factor 180 (wcrf180). The accession number is AL050089. CD9 refers to CD9 antigen (p24), also known as motility related protein 1 (MRP1). The accession number is M38690. PTPN7 refers to protein tyrosine phosphatase non-receptor type 7, with accession number M64322. CDC42 refers to cell division cycle 42, also known as GTP binding protein, 25kDa (G25K). The accession number is M35543. FABP5 refers to fatty acid binding protein 5, with accession number M94856. NAB2 refers to NGFI-A (nerve growth factor induced clone A) binding protein 2, also known as EGR1 binding protein 2. The accession number is X70991. SDR1refers to short-chain dehydrogenase/reductase 1, with accession number AF061741.

In a preferred embodiment, determining the level of a DM1-specific gene product comprises determining the level of said product in an isolated sample from a subject suspected of (developing) DM1 relative to the amount of said DM1-specific gene product in a control sample. Suitable control samples include biological samples isolated from healthy subjects or from patients suffering from Diabetes Mellitus type 2 (DM2). It is also possible to determine the level of a DM1-specific gene product in a sample relative to the level of a housekeeping gene product in the same sample. Generally speaking, housekeeping genes are constitutively expressed genes which serve to maintain cellular function. As such, they are presumed to produce the minimally essential transcripts necessary for normal cellular physiology. With the advent of microarray technology, it has recently become possible to identify at least a "starter set" of housekeeping genes, as exemplified by the work of Velculescu *et al*. (Nat. Genet 23: 387-388, 1999) who examined the expression of 7,000 full-length genes in 11 different human tissues, both adult and fetal, to determine the suite of transcripts that were commonly expressed throughout human development and in different tissues. They identified 535 transcripts via microarray hybridization as likely candidates for housekeeping genes, also referred to as "maintenance" genes.

In addition, other cell types from the subject may sometimes be used as control sample in a method of the invention. Preferably however, a control sample comprises (a defined amount of) an isolated DM1-specific gene product, such as a vector with a DM1-specific gene, purified RNA of a DM1-specific gene, or protein encoded by a DM1-specific gene.

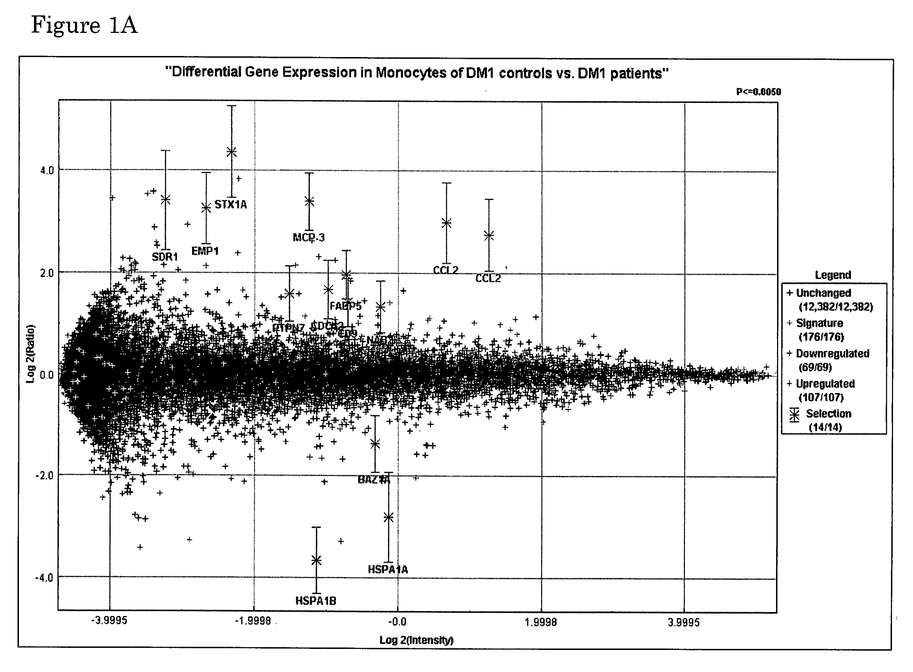

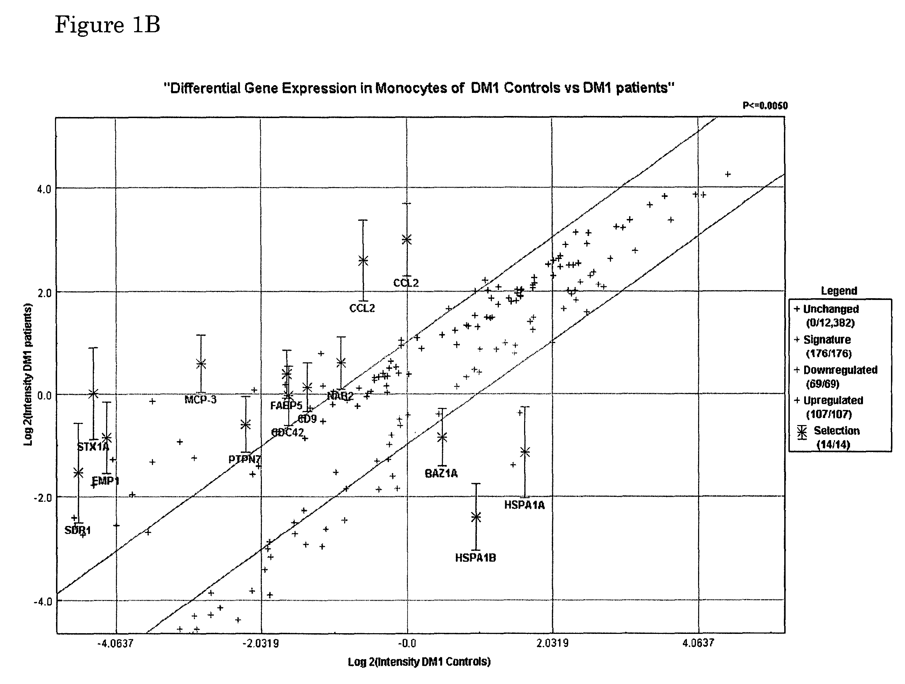

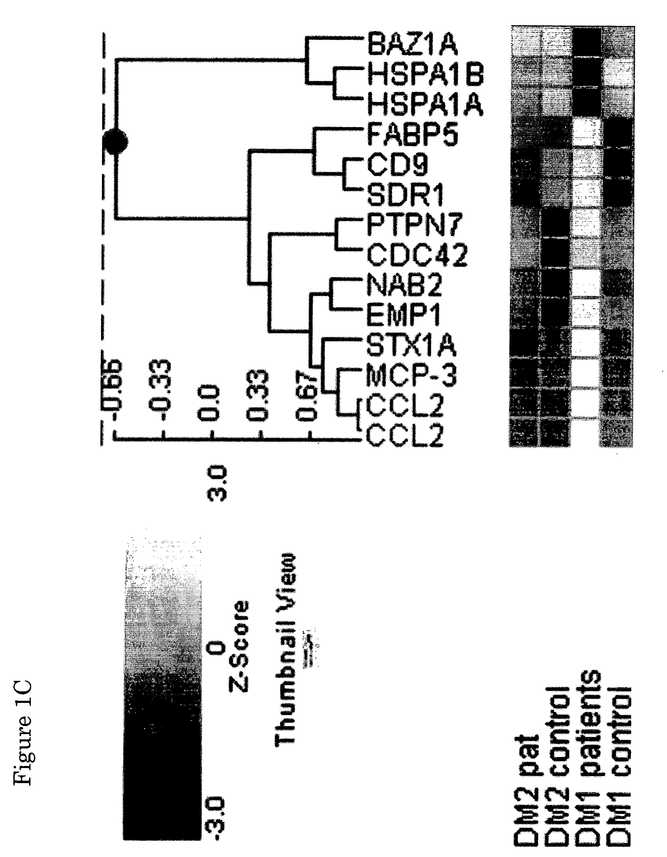

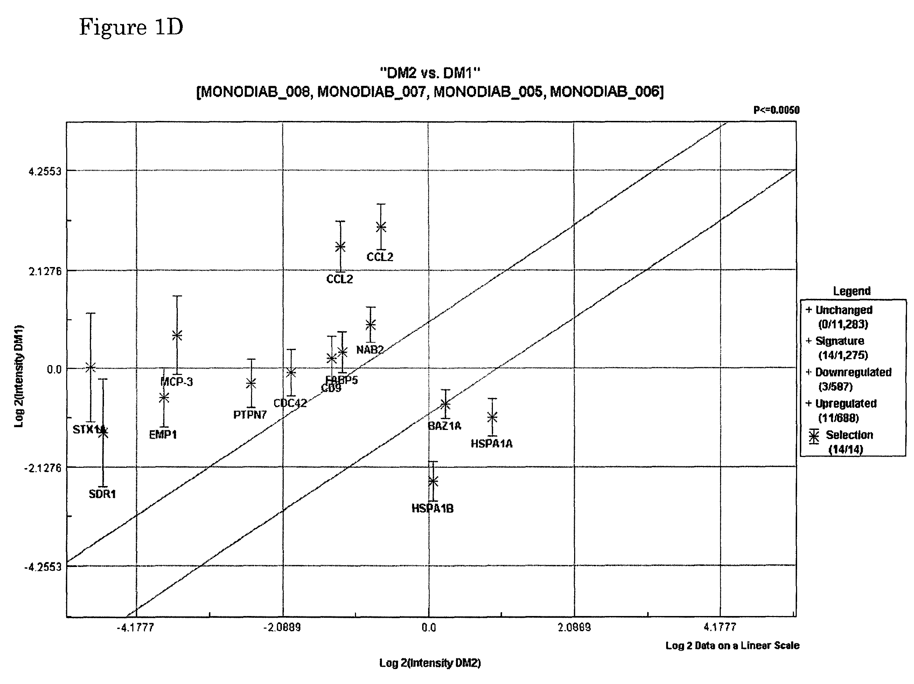

In one embodiment of the invention, the level of a DM1-specific gene product is determined at the DNA or RNA level, preferably at the mRNA level. As is exemplified in the Detailed Description, the expression profile of human monocytes isolated from a DM1 subject was compared to the expression profile in monocytes of healthy subjects or DM2 patients using a U95Av2 GeneChip microarray from Affymetrix. The U95Av2 GeneChip contains over 12,600 transcripts of known genes and expressed sequence tags (ESTs). Surprisingly, it was found that the expression level of 13 human genes was specifically altered in DM1 patients. The term '"DM1-specific gene product" as used herein refers to a product (nucleic acid sequences as well as proteinaceous substances) which is encoded by one of these 13 genes. The expression level of three genes (HSPA1A, HSPA1B and BAZ1A) was reduced in DM1 patients, whereas the expression of nine genes (CCL2, MCP-3, EMP1, STX1A, CD9, PTPN7, CDC42, FABP5, NAB2 and SDR1) was elevated in DM1 patients. For example, Fig. XX shows that CCL2 is highly expressed in DM1 patients, and that this expression is elevated (up-regulated) when compared to age-matched DM1 controls. SDR1 is only expressed at a low level in DM1 patients, but even lower in controls. In contrast, the expression of HSPA1A and HSPA1B is reduced in DM1 patients when compared to controls. Fig. 1 shows that the altered expression level of the 13 genes is not observed in DM2 patients nor in healthy subjects serving as age-matched controls for DM2 patients. Thus, evaluation of the expression level of at least one of these 13 DM1-specific genes ("DM1-markers") allows to discriminate between DM1 and DM2, because an up-regulation of CCL2, MCP-3, EMP1, STX1A, CD9, PTPN7, CDC42, FABP5, NAB2 or SDR1, or a down-regulation of HSPA1A, HSPA1B or BAZ1A expression is only found in DM1 patients and not in DM2 patients or in healthy subjects. Herewith, we have provided diagnostic markers for DM1.
Detection of a DM1-specific nucleic acid can be achieved by conventional techniques well known in the art. These include PCR techniques for detecting DM1-specific DNA sequences, and reverse transcriptase (RT) PCR techniques for detecting DM1-specific RNA sequences. In one embodiment, a DM1-specific gene product is detected using a nucleic acid amplification assay, preferably a PCR assay, using a set of nucleic amplification primers capable of specifically amplifying said gene product. More preferred, said PCR assay is a real-time quantitative PCR (RQ-PCR) assay. The simplest RQ-PCR technique is based on detection of PCR products by the DNA-intercalating dye SYBR Green I. Other suitable RQ-PCR analyses involve those using fluorochrome-labeled sequence-specific probes including hydrolysis probes or hybridisation probes.
In a preferred embodiment of the invention, the increase in the CCL2, MCP-3, EMP1, STX1A, CD9, PTPN7, CDC42, FABP5, NAB2 and/or SDR1 mRNA level is at least two-fold compared to the level of said mRNA in a sample of a healthy subject or a subject suffering from DM2. Likewise, the decrease in the HSPA1A, HSPA1B and/or BAZ1A mRNA level is at least two-fold compared to the level of said mRNA in a sample of a healthy subject or a subject suffering from DM2. More preferred, the altered expression of either one of these DM1-specific genes is more than three-fold, or even more than four-fold, or even higher than that, when compared to control.

A method of the invention is easily practiced in routine laboratory settings. DM1-specific gene products according to the invention can be analysed in various types of cells, including monocytes, dendritic cells, T cells, granulocytes, natural killer T cells and all other (natural) killer cells. All that is required is obtaining or isolating a sample from the subject, preferably a peripheral blood sample, isolating the cell type of interest (preferably monocytes), optionally preparing an extract of the sample (e.g. nucleic acid extract or a total cell lysate) and determining the level of a DM1-specific gene product in the sample or in an extract thereof. The level can be determined by contacting a cellular component of the sample with at least one reagent specifically reactive with a DM1-specific gene product and, optionally, with at least one reagent specifically reactive with a housekeeping gene product. The reagent or reagent(s) may be labeled and they may be immobilized on a solid support, for example a glass, nylon or nitrocellulose solid support. Also provided are reagents, such as nucleotide probes and antibodies specifically reactive with a DM1-specific gene product or with a fragment thereof.

In another embodiment, a nucleic acid extract of a sample isolated from a subject is contacted with at least one nucleotide probe comprising a sequence that hybridises to a nucleotide region encoding a DM1-specific gene and, optionally, with at least one nucleotide probe comprising a sequence that hybridises to a nucleotide region encoding a housekeeping gene. Said at least one nucleotide probe may be immobilized on a solid support, for example in an array format. Examples of suitable nucleotide probes comprise DNA, RNA or cDNA probes. The nucleic acid extract may contain nucleic acids with a detectable label, e.g. biotinylated cRNA.
Different types of DNA microarrays for use in a method of the invention can be prepared according to methods known in the art, e.g. arrays based on standard microscopic glass-slides on which cDNAs or long oligonucleotides (typically 70-80-mers) have been spotted. An other type is based on photolithographic techniques to synthesize 25-mer oligonucleotides on a silicon wafer and constitutes the patented Affymetrix technology.

In a further embodiment, a method to diagnose or screen for DM1 in a subject comprises determining the level of at least one DM1-specific gene product at the protein level. To this end, an extract is prepared from a sample isolated from a subject which allows the specific detection of a protein, for instance by preparing a total cell lysate in the presence of a chaotropic agent such as a detergent or a salt. A sample (comprising at least one cell) from the subject may be pretreated prior to preparing an extract to improve detection of a DM1-specific gene product. For example, some proteins including CCL2 and MCP-3 are known to be rapidly secreted following their synthesis. Pretreatment with a protein secretion inhibitor (e.g. monensin) can thus be used to accumulate a DM1-specific protein within the cell cytoplasm and therewith increase the content of one (or more) DM1-specific gene products in an extract. An extract of a sample can subsequently be contacted with a specific binding partner of at least one protein (or fragment thereof) encoded by a DM1-specific gene under conditions that allow formation of a complex between said binding partner and said protein and detecting the complex formation. The amount of complex formed is indicative of the amount of said DM1-specific protein present in the sample extract. Therefore, it is advantageous to use a binding partner that is provided with a detectable label, such as a fluorochrome, radioactive label, enzyme etc. A well-known method known in the art to specifically detect a protein involves the immunological detection of a protein using a specific (monoclonal or polyclonal) antibody or fragment thereof (e.g. single chain Fv) as a specific binding partner. Monoclonal antibodies may be obtained by well-established methods, e.g. as described in A. Johnstone and R. Thorpe, Immunochemistry in Practice, 2nd. Ed., Blackwell Scientific Publications, 1987, pp. 35-43. When prepared by recombinant DNA techniques, the antibody may be produced by cloning a DNA sequence coding for the antibody or a fragment thereof into a suitable cell, e.g. a microbial, plant, animal or human cell, and culturing the cell under conditions conducive to the production of the antibody or fragment in question and recovering the antibody or fragment thereof from the culture. Possible strategies for the preparation of cloned antibodies are discussed in, for instance, L. Riechmann et al., Nature 332, Mar. 24, 1988, p. 323 ff., describing the preparation of chimeric antibodies of rat variable regions and human constant regions; M. Better et al., Science 240, May 20, 1988, p. 1041 ff., describing the preparation of chimeric mouse-human Fab fragments; A. Sharra and A. Pluckthun, Science 240, May 20, 1988, pp. 1038-1040, describing the cloning of an immunoglobulin Fv fragment containing antigen-binding variable domains; and E. S. Ward et al., Nature 341, Oct. 12, 1989, pp. 544-546, describing the cloning of isolated antigen-binding variable domains ("single-domain antibodies").

In one aspect of the invention, a method is provided to diagnose or screen for DM1 in a subject wherein said method comprises the immunological detection of at least one, preferably at least two, more preferably at least three DM1-specific protein(s) in a biological sample isolated from said subject, wherein said protein is encoded by a gene selected from the group comprising CCL2, MCP-3, HSPA1A, HSPA1B, EMP1, STX1A, BAZ1A, CD9, PTPN7, CDC42, FABP5, NAB2 and SDR1. Immunological detection is a common tool used in discovering protein expression patterns, isolating proteins from cellular extracts and in screening cells and extracts for specific proteins. The immunological detection in a method of the invention can be performed according to well known assays, including Western blotting, immunoprecipitation assays, enzyme-linked immunosorbant assays (ELISA), dot-blot assays and chip-based assays. Detailed practical information regarding the immunological detection of proteins can be found in "Antibodies: A Laboratory Manual " by Ed Harlow and David Lane (Editors) ISBN: 0879695447, Publisher: Cold Spring Harbor. In a specific aspect of the invention, a DM1-ELISA test is provided which is based on the principle of a solid phase enzyme-linked immunosorbant assay. As an example, a CCL2-ELISA test is described. The assay system utilizes a monoclonal antibody directed against an antigenic determinant of CCL2 that is used for solid phase immobilization (e.g. in microtiter wells). A goat antibody conjugated to horseradish peroxidase (HRP) reactive with a different antigenic determinant of CCL2 is present in an antibody-enzyme conjugate solution. The test sample (e.g. cell lysate) is allowed to react simultaneously with the two antibodies, resulting in the CCL2 molecule being sandwiched between the solid phase and enzyme-linked antibodies. After a 1 hour incubation at room temperature, the wells are washed with water to remove unbound labelled antibodies. A solution of HRP-substrate, for instance 3,3',5,5'-tetramethylbenzidine (TMB), is added and incubated for 20 minutes, resulting in the development of a blue colour. The colour development is stopped with the addition of a stop solution (e.g. 1 N HCl) changing the colour to yellow. Absorbance is measured spectrophotometrically at 450 nm. The concentration of CCL2 is directly proportional to the colour intensity of the test sample. An increase in the concentration of CCL2 in a sample is indicative of DM1. [Of course it is preferred to include a standard curve of known amounts of the CCL2 protein in order to quantitate the amount of CCL2 present. Furthermore, the detection of a protein which is not subject to a change in DM1 patients (for example a housekeeping enzyme) may be used as internal control for the amount of protein assayed and/or as a control between different samples.]

In a further embodiment, a test kit is provided for diagnosing or screening for Diabetes Mellitus Type 1 (DM1) in a subject comprising at least one reagent specifically reactive with a DM1-specific gene product selected from the group comprising CCL2, MCP-3, HSPA1A, HSPA1B, EMP1, STX1A, BAZ1A, CD9, PTPN7, CDC42, FABP5, NAB2 and SDR1. Said kit optionally further comprises at least one reagent specifically reactive with a housekeeping gene product. A suitable reagent can be an antibody or fragment thereof, or a nucleotide probe, specifically reactive with a DM1-specific gene product. Said reagent may be immobilized on a solid support, preferably a glass, nylon or nitrocellulose solid support. The physical shape of the solid support is not critical, although some shapes may be more convenient than others for the present purpose. Thus, the solid support may be in the shape of a plate, e.g. a microtiter plate, or a paper strip, dipstick, membrane (e.g. a nylon membrane or a cellulose filter) or solid particles (e.g. latex beads). Furthermore, a kit preferably also comprises a defined amount of one or more DM1-specific gene products which can serve as a control sample. A vector with a DM1-specific gene, purified RNA of a DM1-specific gene, or protein encoded by a DM1-specific gene can be used as a quantitative control.

For example, a DM1 test kit is provided comprising an array of nucleotide probes comprising at least 10 nucleotide bases in length, wherein at least one probe hybridises to a fragment of at least one DM1-specific gene selected from the group comprising CCL2, MCP-3, HSPA1A, HSPA1B, EMP1, STX1A, BAZ1A, CD9, PTPN7, CDC42, FABP5, NAB2 and SDR1, and optionally, wherein at least one probe hybridises to a fragment of at least one housekeeping gene. Of course, the different probes should be specific for the different DM1-specific genes. Said array of nucleotide probes is for instance arranged on a solid support in multiple discrete regions of distinct nucleic acid strands.

In another example, a kit according to the invention to diagnose or screen for DM1 comprises one or more antibodie(s) specifically reactive with a DM1-specific protein. Said antibodies can be labelled to aid in detection of an antibody-antigen complex. The label substance for the reagents is preferably selected from the group consisting of enzymes, coloured or fluorescent substances and radioactive isotopes.

Examples of enzymes useful as label substances are peroxidases (such as horseradish peroxidase), phosphatases (such as acid or alkaline phosphatase), β-galactosidase, urease, glucose oxidase, carbonic anhydrase, acetylcholinesterase, glucoamylase, lysozyme, malate dehydrogenase, glucose-6-phosphate dehydrogenase, β-glucosidase, proteases, pyruvate decarboxylase, esterases, luciferase, etc. Enzymes are not in themselves detectable but must be combined with a substrate to catalyse a reaction the end product of which is detectable. Thus, a substrate may be added after contacting the support with the labelled reagent, resulting in the formation of a coloured or fluorescent substance. Examples of substrates which may be employed according to the invention include hydrogen peroxide/tetramethylbenzidine or chloronaphthole or o-phenylenediamine or 3-(p-hydroxyphenyl) propionic acid or luminol, indoxyl phosphate, p-nitrophenylphosphate, nitrophenyl galactose, 4-methyl umbelliferyl-D-galactopyranoside, or luciferin. Alternatively, the label substance may comprise coloured or fluorescent substances, Europium, gold particles, coloured or fluorescent latex particles, dye particles, fluorescein, phycoerythrin or phycocyanin. Radioactive isotopes which may be used for the present purpose may be selected from I-125, I-131, H-3, P-32, P-33 and C-14.

Preferably, a kit further comprises standard amounts of (lyophilised) DM1-specific protein and, optionally, other reagents required for immunological detection of a protein such as a binding buffer, enzyme substrate, stop solution, and the like. DM1-specific protein may be obtained using recombinant expression of a nucleic acid sequence encoding said DM1-specific protein in a host cell, preferably followed by purification of the protein.

A method of the invention, a kit as defined above or an array of probes is advantageously used in the diagnosis or screening for increased risk of developing DM1, to discriminate between DM1 and DM2 in a subject or to evaluate the risk of developing or to diagnose LADA in a subject, preferably a human subject.

### Example

### Experimental Procedures

### Subjects

Peripheral blood mononuclear cells (PBMCs) were isolated from heparinized peripheral blood using Ficoll-Paque (Amersham Biosciences, Uppsala, Sweden) density gradient centrifugation, resuspended in RPMI1640 medium containing HEPES and ultraglutamin supplemented with 10% fetal calf serum and 10% DMSO, and stored at -180 °C providing a bank for experiments. This study was approved by the Medical Ethical Committee of the Erasmus MC, Rotterdam, The Netherlands. All subjects provided written informed consent prior to participation.

At the start of an experiment, the PBMCs were quickly thawed and pooled into patient samples or control samples to minimize individual variations. 8-9 recent onset type 1 Diabetes Mellitus (DM1) patients were pooled into DM1 samples (3 different pools; mean age 14.4 years), 7-8 healthy controls for DM1 patients (CoDM1; age-matched) into CoDM1 sample (3 different pools), 7 type 2 Diabetes Mellitus (DM2) patients into a DM2 sample (mean age 55.9 years), and 7 healthy controls for DM2 patients (CoDM2; age-matched) into a CoDM2 sample.

The pooled cell samples were labeled with anti-CD14 magnetic beads (Miltenyi Biotec GmbH, Bergisch Gladbach, Germany) for 20 minutes at 4 °C and separated into CD14+ and CD 14- fractions using the autoMACS (Miltenyi Biotec), program positive selection sensitive. The CD14+ fraction represents the monocytes with a purity of at least 94%.

### RNA isolation

Total RNA was extracted from the monocytes with the use of a RNeasy Mini kit and QIAshredders from Qiagen (Westburg, Leusden, The Netherlands) according to the manufacturer's instructions. The integrity of the RNA was tested on 1.2% formaldehyde containing agarose gels.

### Target preparation

DNA complementary to the total RNA samples was synthesized from 4-5 µg of total RNA using an Superscript double-Stranded Synthesis kit from Invitrogen (Breda, The Netherlands) and a T7-(dT)24 primer (GenSet Oligos, Paris, France) according to the manufacturer's instructions. The cDNA served as a template for in vitro transcription reaction (37 °C for 5 h) using T7 RNA polymerase and biotinylated ribonucleotides employing an Enzo BioArray High Yield RNA transcript labeling kit (Enzo Life Sciences, Farmingdale, New York, USA). The cDNA and cRNA was purified using the GeneChip Sample Cleanup module (Affymetrix, Santa Clara, California, USA). The cRNA was quantified by spectrophotometric methods. An adjusted cRNA yield was calculated to reflect carryover of unlabeled total RNA.

### Hybridization and Staining

Twenty micrograms of biotinylated cRNA was randomly fragmented by heat and alkaline treatment. To check the quality of the procedure 5 µg cRNA was hybridized to a Test3 microarray (Affymetrix). Subsequently 10 µg of cRNA was hybridized to an U95Av2 microarray (Affymetrix; HG-U95Av2 GeneChip) for 16 h at 45 °C. The U95Av2 GeneChip contains 12,600 transcripts of known genes and expressed sequence tags (ESTs) that are characterized on their function- or disease-association. After washing and staining with PE-conjugated streptavidin, the microarrays were scanned in an HP/Affymetrix scanner at 570 nm using a krypton/argon laser.

### Data analysis

Image analysis was performed with Microarray Suite, version 5.0 (Affymetrix) using the control sample as baseline. To facilitate comparison between samples and experiments, the trimmed mean signal of each microarray was scaled to a target intensity of 2500. The expression profiles of DM1 and DM2 were compared to expression profiles of the CoDM1 and CoDM2, respectively.
Metric files from comparison analysis were exported to Microsoft Excel software package for further filtering and analysis. Genes (or ESTs) called 'significantly changed' were those that possessed 1) a reliably detectable signal in control and/or patient sample and 2) were determined by the statistical algorithm to be changed 2-fold or greater. To increase stringency, genes meeting the above criteria were filtered further to include only those that 1) were significantly changed between DM1 and CoDM1 but not changed between DM2 and CoDM2 or changed in the opposite direction (increased in DM1 versus decreased in DM2, and vice versa) and 2) were consistently differently expressed between DM1 and CoDM1 in all three experiments. The expression profiles were also exported to the Rosetta Resolver Expression Data Analysis System (Rosetta Inpharmatics, Kirkland, Washington, USA) to perform cluster analysis. To increase confidence, the three DM1 profiles and three CoDM1 profiles were combined in an error weighted fashion into a single DM1 experiment and CoDM1 experiment, respectively. The data of these DM1 and CoDM1 experiments were used to create an intensity-based ratio experiment. The genes that were changed at least 4-fold and had a p≤0.01 in this ratio experiment were grouped together as a bioset. We then clustered data of the DM1-, CoDM1, DM2, and CoDM2 experiments restricted to the genes in the biosets employing the agglomerative clustering algorithm. The results of this 2D cluster was displayed in a heatmap in which each cell represents a log(ratio). This heatmap was used to select DM1-specific genes, that is genes that were up- or downregulated in DM1 patients only, not in DM2 patients nor in CoDM1 and CoDM2 (see Figures 1 A to D).

### Legends

Figure 1: Microarray analysis of DM1 patients, DM2 patients and their sex- and age-matched controls. Purified monocytes were pooled and analyzed for gene expression using DNA microarrays.
A) Scatter plot of the ratio (Y-axis) versus the intensity (X-axis) of genes differentially expressed in DM1 patients as compared with sex- and age-matched controls is shown. Expression levels were averaged (weighted on basis of error) across the three replicate experiments.
B) Scatter plot of intensity of genes in DM1 patients (Y-axis) versus sex- and age-matched DM1 controls (X-axis) is shown. Expression levels were averaged (weighted on basis of error) across the three replicate experiments. The selected DM1-specific genes are indicated with an asterisk.
C) Relative expression of the selected DM1-specific genes across DM1 patients, DM1 controls, DM2 patients, and DM2 controls are shown in a heatmap. Intensities were normalized across all intensity experiments and expressed on a gray scale by use of the software Rosetta Resolver. The increase (white) and decrease (black) in expression level relative to the median value are shown for the 4 experiments. The genes were also clustered using agglomerative clustering algorithm. Rows represent the relative intensity in the four groups. Columns represent the genes. Each cell in the heatmap represents a Z-score.
D) Scatter plot of the average intensities in DM1 patients (Y-axis) versus the intensity in DM2 patients (X-axis) is shown. Genes were selected on the basis of a significant change in expression between DM1 and DM2/controls. Expression levels in DM1 patients were averaged (weighted on basis of error) across the three replicate experiments.

## Claims

1. A method to diagnose or screen for Diabetes Mellitus type 1 (DM1) in a subject, preferably a human subject, said method comprising determining the level of at least one, preferably at least two, more preferably at least three, most preferred at least four, DM1-specific gene product(s) in a biological sample isolated from said subject, preferably peripheral blood monocytes, wherein said DM1-specific gene is selected from the group comprising CCL2, MCP-3, HSPA1A, HSPA1B, EMP1, STX1A, BAZ1A, CD9, PTPN7, CDC42, FABP5, NAB2 and SDR1.

2. A method according to claim 1, wherein determining the level comprises determining the level of said at least one DM1-specific gene product in said isolated sample relative to the level of said at least one DM1-specific gene product in a control sample, preferably a biological sample isolated from a healthy subject or from a subject suffering from Diabetes Mellitus type 2 (DM2).

3. A method according to claim 1 or 2, wherein the level of said gene product is determined at the DNA or RNA level, preferably the mRNA level.

4. A method according to claim 3, wherein the mRNA level of CCL2, MCP-3, EMP1, STX1A, CD9, PTPN7, CDC42, FABP5, NAB2 and/or SDR1 in a sample of said subject is at least two-fold higher and/or wherein the mRNA level of HSPA1A, HSPA1B and/or BAZ1A is at least two-fold lower compared to the presence of said mRNA in a sample of a healthy subject or a subject suffering from Type 2 Diabetes Mellitus (DM2).

5. A method according to any one of claims 1-4, wherein determining the level is performed by contacting a nucleic acid extract of said sample with at least one probe comprising a sequence that hybridises to a nucleotide region encoding a DM1-specific gene and, optionally, with at least one probe comprising a sequence that hybridises to a nucleotide encoding a housekeeping gene, wherein said at least one nucleotide probe is preferably immobilized on a solid support.

6. A method according to claim 5, wherein said nucleotide probes comprise DNA, RNA or cDNA and/or wherein said nucleic acid extract comprises nucleic acids with a detectable label.

7. A method according to claim 1 or 2, wherein the level of said gene product is determined at the protein level.

8. A method according to claim 7, wherein determining the level is performed by contacting a protein extract of said sample with a specific binding partner of at least one protein encoded by a DM1-specific gene under conditions that allow formation of a complex between said binding partner and said protein and detecting complex formation.

9. A kit for diagnosing or screening for Diabetes Mellitus Type 1 (DM1) in a subject comprising at least one reagent specifically reactive with a DM1-specific gene product selected from the group comprising CCL2, MCP-3, HSPA1A, HSPA1B, EMP1, STX1A, BAZ1A, CD9, PTPN7, CDC42, FABP5, NAB2 and SDR1, optionally further comprising at least one reagent specifically reactive with a housekeeping gene product and/or a defined amount of a DM1-specific gene product.

10. A kit according to claim 9, wherein said reagent comprises an antibody or fragment thereof, or a nucleotide probe.

11. A kit according to claim 10 or 11, wherein said at least one reagent is immobilized on a solid support, preferably a glass, nylon or nitrocellulose solid support.

12. An array of nucleotide probes comprising at least 10 nucleotide bases in length, wherein at least one probe hybridises to a fragment of at least one DM1-specific gene selected from the group comprising CCL2, MCP-3, HSPA1A, HSPA1B, EMP1, STX1A, BAZ1A, CD9, PTPN7, CDC42, FABP5, NAB2 and SDR1, and optionally, wherein at least one probe hybridises to a fragment of at least one housekeeping gene.

13. Use of a method according to any one of claims 1-9, a kit according to any one of claims 10-13 or an array according to claim 14 to diagnose or screen for increased risk of developing Diabetes Mellitus type 1 (DM1), to discriminate between DM1 and DM2 in a subject or to evaluate the risk of developing or to diagnose latent autoimmune diabetes of adulthood (LADA) in a subject, preferably a human subject.
